(19) 

**Europäisches Patentamt**
**European Patent Office**
**Office européen des brevets**

(11) **EP 4 553 468 A1**

(12) 

# EUROPEAN PATENT APPLICATION
published in accordance with Art. 153(4) EPC

(43) Date of publication:
**14.05.2025 Bulletin 2025/20**

(51) International Patent Classification (IPC):
**G01M 3/02** $^{(2006.01)}$

(21) Application number: **23859681.1**

(52) Cooperative Patent Classification (CPC):
**G01M 3/02**

(22) Date of filing: **22.02.2023**

(86) International application number:
**PCT/JP2023/006375**

(87) International publication number:
**WO 2024/047907 (07.03.2024 Gazette 2024/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.08.2022 JP 2022138048**

(71) Applicant: **Mitsubishi Heavy Industries, Ltd.**
**Tokyo 100-8332 (JP)**

(72) Inventors:
• **KAMIHARA, Nobuyuki**
  **Tokyo 100-8332 (JP)**

• **ARAKAWA, Yoshiaki**
  **Yokohama-shi, Kanagawa 220-8401 (JP)**
• **INUI, Masayuki**
  **Tokyo 100-8332 (JP)**
• **SHIBUYA, Hidekazu**
  **Tokyo 100-8332 (JP)**
• **KAWAZOE, Kohei**
  **Tokyo 100-8332 (JP)**
• **MORITAKE, Takayuki**
  **Tokyo 100-8332 (JP)**

(74) Representative: **Studio Torta S.p.A.**
**Via Viotti, 9**
**10121 Torino (IT)**

(54) **GAS LEAKAGE MONITORING METHOD, GAS LEAKAGE MONITORING SYSTEM, AND GAS LEAKAGE MONITORING PROGRAM**

(57) A gas leakage monitoring method includes detecting a gas leaking from a field, by using a gas detection device mounted on a first UAV, and acquiring location information of the gas by using a location measurement device mounted on the first UAV. Thereafter, the gas leakage monitoring method includes moving a second UAV mounted with a concentration measurement device including a contact gas sensor to above a gas leakage location corresponding to the location information, and measuring a concentration of the gas at the gas leakage location. The gas leakage monitoring method includes calculating a leakage amount of the gas based on a volume of the gas detected by the gas detection device and the concentration measured by the concentration measurement device.

FIG. 2

EP 4 553 468 A1

## Description

TECHNICAL FIELD

The present disclosure relates to a gas leakage monitoring method and a gas leakage monitoring system.

This application claims the priority of Japanese Patent Application No. 2022-138048 filed on August 31, 2022, the content of which is incorporated herein by reference.

BACKGROUND

As a technique for monitoring gas leakage in a field, it is known that the field is photographed using an image capturing device such as a camera and a leaking gas is sensed based on captured image data. For example, Patent Document 1 discloses a black exhaust sensing system for sensing a gas (black exhaust) in a field by using optical flow estimation. In this black exhaust sensing system, two consecutively captured time-series images of the black exhaust emitted from a flare stack is used to obtain local velocity vectors within the images, which are caused by movement of the gas, from optical flow estimation between the two images. Of the velocity vectors thus obtained, the vector whose magnitude and direction are within a predetermined range is extracted as a velocity vector indicating the movement of the black exhaust.

Citation List

Patent Literature

Patent Document 1: JP4266535B

SUMMARY

Technical Problem

When a gas leaking from a field is to quantitatively be evaluated, a leakage amount M can be calculated based on a volume V and a concentration p of the gas (specifically, the leakage amount M can be obtained as a product of the volume V and the concentration p). The volume V of the gas can be estimated based on, for example, the range of the gas captured in the image data as in Patent Document 1 described above. However, in order to obtain the concentration p of the gas with sufficient accuracy, it is necessary to directly measure the gas at a gas leakage site by using a contact sensor, for example. Therefore, when the field to be monitored is large and it is difficult to identify in advance a location where gas leakage may occur, it is necessary to place contact sensors in many locations where gas leakage may occur, which is not realistic.

At least one embodiment of the present disclosure was made in view of the above issue, and an object of at least one embodiment of the present disclosure is to provide a gas leakage monitoring method, a gas leakage monitoring system, and a gas leakage monitoring program, which are capable of quantitatively evaluating a gas leaking from a field.

Solution to Problem

In order to solve the above-described problems, a gas leakage monitoring method according to at least one embodiment of the present disclosure, includes: a detection step of detecting a gas leaking from a field, by using a gas detection device mounted on a first UAV; a location information acquisition step of acquiring location information of the gas by using a location measurement device mounted on the first UAV; a movement step of moving a second UAV mounted with a concentration measurement device including a contact gas sensor to above a gas leakage location corresponding to the location information; a concentration measurement step of measuring a concentration of the gas at the gas leakage location by using the concentration measurement device; and a leakage amount calculation step of calculating a leakage amount of the gas based on a volume of the gas detected by the gas detection device and the concentration measured by the concentration measurement device.

In order to solve the above-described problems, a gas leakage monitoring system according to at least one embodiment of the present disclosure, includes: a first UAV mounted with a gas detection device for detecting a gas leaking from a field and a location measurement device for acquiring location information of the gas; a second UAV movable based on the location information and mounted with a concentration measurement device for measuring a concentration of the gas; and a leakage amount calculation device for calculating a leakage amount of the gas based on a volume of the gas detected by the gas detection device and the concentration measured by the concentration measurement device.

In order to solve the above-described problems, a gas leakage monitoring program according to at least one embodiment of the present disclosure, causes a computer to execute: a detection step of detecting a gas leaking from a field, by using a gas detection device mounted on a first UAV; a location information acquisition step of acquiring location information of the gas by using a location measurement device mounted on the first UAV; a movement step of moving a second UAV mounted with a concentration measurement device including a contact gas sensor to above a gas leakage location corresponding to the location information; a concentration measurement step of measuring a concentration of the gas at the gas leakage location by using the concentration measurement device; and a leakage amount calculation step of calculating a leakage amount of the gas based on a volume of the gas detected by the gas detection device and the concentration measured by the concentration measurement device.

Advantageous Effects

According to at least one embodiment of the present disclosure, it is possible to provide a gas leakage monitoring method, a gas leakage monitoring system, and a gas leakage monitoring program, which are capable of quantitatively evaluating a gas leaking from a field.

BRIEF DESCRIPTION OF DRAWINGS

FIG. 1 is a schematic view of a UAV of a gas leakage monitoring system according to an embodiment.
FIG. 2 is a configuration diagram of the gas leakage monitoring system according to an embodiment.
FIG. 3 is a flowchart showing a gas leakage monitoring method according to an embodiment.
FIG. 4 is a schematic view showing, from the side, one mode of measuring a concentration by a concentration measurement device in step S3 of FIG. 3.
FIG. 5 is a modified example of FIG. 4.
FIG. 6 is a schematic view showing, from the side, another mode of measuring the concentration by the concentration measurement device in step S3 of FIG. 3.
FIG. 7 is a schematic view showing, from the side, another mode of measuring the concentration by the concentration measurement device in step S3 of FIG. 3.
FIG. 8 is a graph showing an example of a concentration distribution with respect to a height.

DETAILED DESCRIPTION

Some embodiments of the present invention will be described below with reference to the accompanying drawings. It is intended, however, that unless particularly identified, dimensions, materials, shapes, relative positions and the like of components described or shown in the drawings as the embodiments shall be interpreted as illustrative only and not intended to limit the scope of the present disclosure.

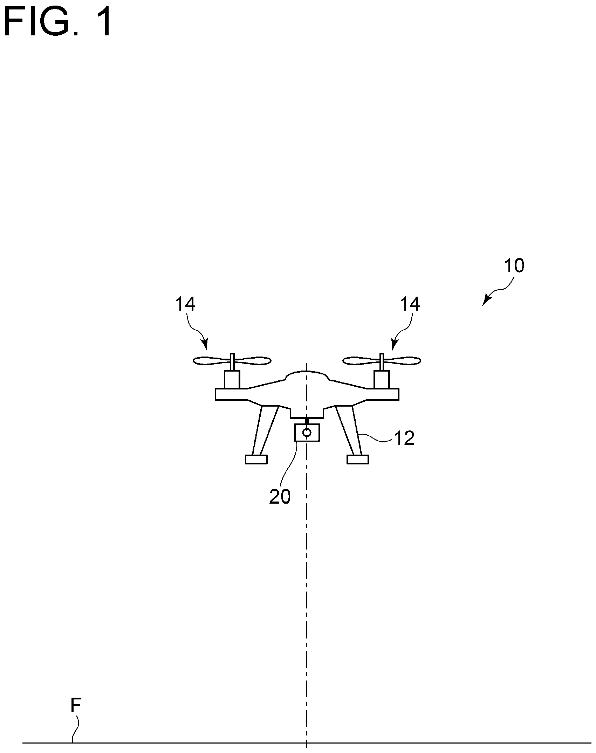

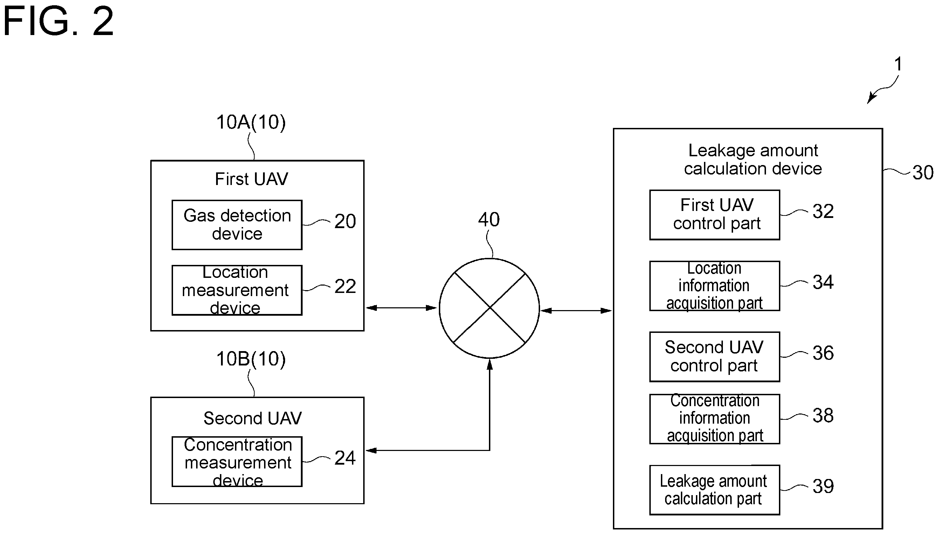

FIG. 1 is a schematic view of a UAV of a gas leakage monitoring system 1 according to an embodiment, and FIG. 2 is a configuration diagram of the gas leakage monitoring system 1 according to an embodiment.

The gas leakage monitoring system 1 is a system for monitoring a gas G (for example, leakage or outflow of the gas from a facility) in a field F by using a UAV 10 (Unmanned Aerial Vehicle). The type of gas G to be monitored is not particularly limited. Hereinafter, as an example, a case in which carbon dioxide ($CO_2$) gas is to be monitored will be described.

As shown in FIG. 1, the UAV 10 includes a UAV body 12 and a plurality of propellers 14 (typically, at least three propellers 14) attached to the UAV body 12. Each of the plurality of propellers 14 is configured to rotationally be driven by a corresponding one of motors (not shown). A flight height, a flight speed, a flight direction, etc. of the UAV 10 can be controlled by adjusting an electric current value of each motor for driving a corresponding one of the plurality of propellers 14. A signal indicating the electric current value of each motor may be sent to a leakage amount calculation device 30 (see FIG. 2) via a wireless communication network 40, as described later.

The UAV 10 may be provided with at least either of an altimeter (not shown) for measuring a flight height H of the UAV 10 or a speedometer (not shown) for measuring a flight speed v of the UAV 10. At least either of a signal indicating the flight height H of the UAV 10, which is acquired by the altimeter, and/or a signal indicating the flight speed v of the UAV 10, which is acquired by the speedometer, may be sent to the leakage amount calculation device 30 via the wireless communication network 40.

As shown in FIG. 2, the gas leakage monitoring system 1 includes a first UAV 10A, a second UAV 10B, and the leakage amount calculation device 30. The first UAV 10A and the second UAV 10B are each the UAV 10 having the basic configuration shown in FIG. 1, and as described below, are each mounted with various devices according to its function (hereinafter, when collectively referring to the first UAV 10A and the second UAV 10B, they will be referred to as the "UAV 10" as appropriate).

The first UAV 10A is the UAV 10 mounted with a gas detection device 20 and a location measurement device 22. The first UAV 10A can communicate via the wireless communication network 40, and can send and receive various kinds of information including a control signal for realizing a movement route on the field F, a detection result of the gas detection device 20, and a measurement result of the location measurement device 22.

The gas detection device 20 is configured to detect the gas G leaking from the field F, in a state in which the UAV 10 is flying. For example, the gas detection device 20 is an image capturing device capable of capturing an image of the field F below the UAV 10 over a predetermined field of view. In this case, the gas detection device 20 may be an infrared camera including a filter that selectively transmits infrared light of a wavelength (4.3 $\mu$m in the case of $CO_2$ gas) absorbed by the gas G to be monitored. By thus using the infrared camera that selectively filters and images the specific wavelength, only the specific gas G is imaged in image data for a fluid (gas) in the field of view. The image data including the gas G detected by the gas detection device 20 may be sent to the leakage amount calculation device 30 via the wireless communication network 40.

The location measurement device 22 is configured to measure a location of the gas G in the field F, which is detected by the gas detection device 20. The measurement result of the location measurement device 22 is location information including location coordinates in the field F, and the location information may be sent to the leakage amount calculation device 30 (and may also be sent to the second UAV 10B) via the wireless communication network 40. A method for measuring the location by the location measurement device 22 is not limited, but the location measurement device 22 may include, for example, a GPS sensor and acquire GPS location information as the location information.

Since the first UAV 10A is thus mounted with the gas detection device 20 and the location measurement device 22, the gas G in field F is detected by the gas detection device 20 and the location information is acquired by measuring the location of the gas G. The location information obtained in the first UAV 10A is sent to the second UAV 10B or the leakage amount calculation device 30 via the wireless communication network 40.

The second UAV 10B is the UAV 10 mounted with a concentration measurement device 24 for measuring the concentration of the gas G. The second UAV 10B can move to above the location of the gas G (hereinafter, referred to as the "gas leakage location" as appropriate), which is detected by the gas detection device 20, based on the location information obtained in the first UAV 10A. The second UAV 10B may acquire the location information obtained in the first UAV 10A from the leakage amount calculation device 30 or may acquire the location information directly from the first UAV 10B, via the wireless communication network 40.

The concentration measurement device 24 includes a contact sensor, and is configured to measure the concentration of the gas around the concentration measurement device 24. As described later, the second UAV 10B can measure the concentration of the gas G at a predetermined height from the field F by lowering the concentration measurement device 24 from the UAV body 12 or by lowering the UAV body 12 itself mounted with the concentration measurement device 24.

The leakage amount calculation device 30 is configured to calculate a leakage amount of the gas G in the field F by sending and receiving various data to and from the first UAV 10A and the second UAV 10B via the wireless communication network 40, and includes a first UAV control part 32, a location information acquisition part 34, a second UAV control part 36, a concentration information acquisition part 38, and a leakage amount calculation part 39. For example, the leakage amount calculation device 30 has a hardware configuration which is a calculator with a processor (such as CPU or GPU), a storage device (memory device; such as RAM), an auxiliary storage unit, an interface, and the like, and can send and receive the various data to and from the first UAV 10A and the second UAV 10B via a predetermined interface. The processor is configured to process the signal thus received. In addition, the processor is configured to process programs loaded into the storage device. Whereby, various functions of the leakage amount calculation device 30 are realized.

Processing contents in the leakage amount calculation device 30 is implemented as the programs executed by the processor. The programs may be stored in the auxiliary storage unit. When executed, these programs are loaded into the storage device. The processor reads out the programs from the storage device to execute instructions included in the programs.

The first UAV control part 32 is configured to control the first UAV 10A. Specifically, the first UAV control part 32 controls movement such that the gas detection device 20 mounted on the first UAV 10A detects the gas G throughout the entire field F to be monitored, by moving the first UAV 10 on the field F, and when the gas G is detected, controls the location measurement device 22 mounted on the first UAV 10A to measure the location of the gas G.

The location information acquisition part 34 is configured to acquire location information corresponding to the location measured by the location measurement device 22 mounted on the first UAV 10A. The location information acquired by the location information acquisition part 34 may readably be stored in a storage device (not shown).

The second UAV control part 36 is configured to control the second UAV 10B to move to information of the gas leakage location based on the location information acquired by the location information acquisition part 34, and to control the concentration measurement device 24 mounted on the second UAV 10B to measure the concentration of the gas G at the gas leakage location. The movement control of the second UAV 10B by the second UAV control part 36 is performed such that the second UAV 10B is positioned above the gas leakage location on the field F, which is identified based on the location information.

The concentration information acquisition part 38 is configured to acquire data regarding the concentration of the gas G, which is measured by the concentration measurement device 24 mounted on the second UAV 10B. The data regarding the concentration acquired by the concentration information acquisition part 38 may readably be stored in the storage device (not shown).

The leakage amount calculation part 39 is configured to calculate the leakage amount of the gas G detected by the gas detection device 20 mounted on the first UAV 10A. A leakage amount Q is calculated using a volume V and a concentration p of the gas G, as follows.

$$Q = V \times \rho \quad (1)$$

The volume V can be estimated by, for example, evaluating spread of the gas G in the field F, based on the image data captured by the gas detection device 20 which is the image capturing device. As for the concentration p, the data acquired by the concentration information acquisition part 38 as described above is used.

As shown in FIG. 1, the leakage amount calculation device 30 is disposed at a location remote from the first UAV 10A and the second UAV 10B, and can receive data regarding the volume V and the concentration p via the wireless communication network 40. Whereby, in the remote location, it is possible to quantitatively monitor the gas leakage in the field F.

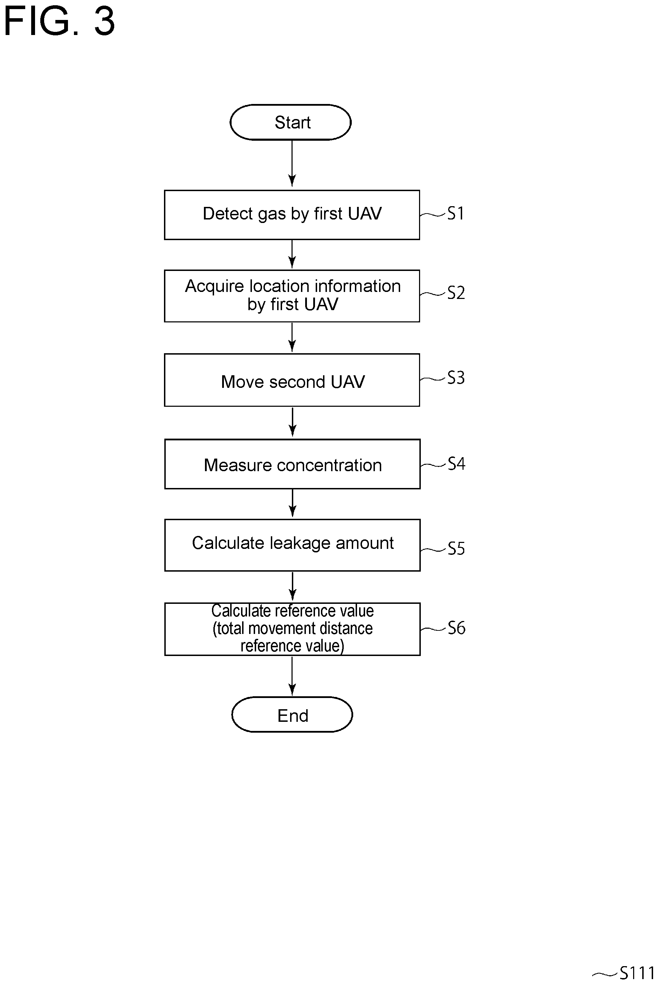

Subsequently, the gas leakage monitoring method using the gas leakage monitoring system 1 having the above configuration will be described. FIG. 3 is a flowchart showing the gas leakage monitoring method according to an embodiment.

First, the first UAV control part 32 detects the gas G in the field F by using the gas detection device 20 mounted on the first UAV 10A, while moving on the field F by the first UAV 10A (step S1: detection step). The movement route of the first UAV 10A is set such that the field F to be monitored is included in the imaging range of the image capturing device which is the gas detection device 20. Further, the detection of the gas G by the gas detection device 20 is performed, for example, by capturing the image of the field F with the gas detection device 20 which is the image capturing device. The detection result by the gas detection device 20 is sent to the leakage amount calculation device 30 via the wireless communication network 40.

Next, the first UAV control part 32 acquires the location information of the gas G detected in step S1, by using the location measurement device 22 mounted on the first UAV 10A (step S2: location information acquisition step). The location information is acquired as three-dimensional coordinates indicating the location of the gas G in the field F, for example. The location information acquired in step S2 is sent to the leakage amount calculation device 30 via the wireless communication network 40, and is acquired by the location information acquisition part 34.

Next, the second UAV control part 36 moves the second UAV 10B based on the location information acquired in step S2 (step S3: movement step). The movement control in step S3 is performed such that the second UAV 10B is positioned above the gas leakage location identified by the location information. For example, if the gas leakage location is identified by three-dimensional coordinates (x, y, z), the second UAV 10B is controlled to hover at a predetermined height at the location identified by coordinates (x, y) when the field F is viewed from above.

Next, the second UAV control part 36 measures the concentration p of the gas G at the gas leakage location by using the concentration measurement device 24 mounted on the second UAV 10B (step S4: concentration measurement step). Since the concentration measurement device 24 includes the contact sensor as described above, at the gas leakage location, the concentration p at the height where the concentration measurement device 24 is located is measured.

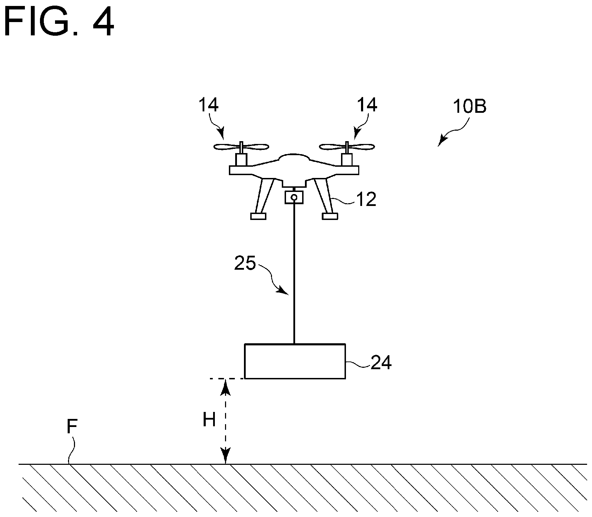

In one mode of step S4 (concentration measurement step), the concentration measurement device 24 may measure the concentration p in a state in which the concentration measurement device 24 is lowered from the second UAV 10B to the predetermined height from the field F (ground surface). Herein, FIG. 4 is a schematic view showing, from the side, one mode of measuring the concentration p by the concentration measurement device 24 in step S3 of FIG. 3. In this mode, the second UAV 10B includes a hoisting and lowering mechanism 25 for hoisting and lowering the concentration measurement device 24 from the UAV body 12. The second UAV control part 36 lowers the concentration measurement device 24 from the UAV body 12 to the predetermined height H from the field F while controlling the flight state of the second UAV 10B such that the second UAV 10B hovers above the gas leakage location. Whereby, the concentration measurement device 24 can measure the concentration p at the predetermined height.

In this mode, after the measurement of the concentration p at the predetermined height H is completed, the concentration measurement device 24 is hoisted by the hoisting and lowering mechanism 25 and stored in the UAV body 12.

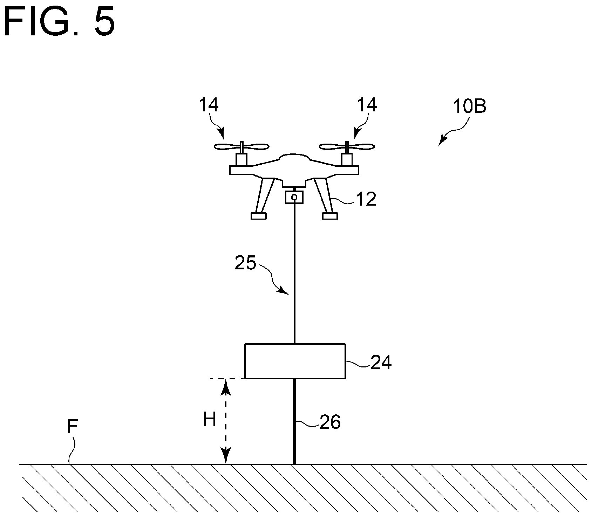

FIG. 5 is a modified example of FIG. 4. In this modified example, an anti-sway mechanism 26 is disposed at the bottom of the concentration measurement device 24. The anti-sway mechanism 26 is configured to contact the field F when the concentration measurement device 24 is effected from the UAV body 12 to the predetermined height H by the hoisting and lowering mechanism 25. In the example of FIG. 5, the anti-sway mechanism 26 is a rod member extending along the vertical direction, one end of the anti-sway mechanism 26 is attached to the bottom of the concentration measurement device 24, and another end of the anti-sway mechanism 26 contacts the field F when the concentration measurement device 24 is at the predetermined height H. Whereby, the concentration measurement device 24 which is lowered from the second UAV 10B to the predetermined height H has its posture stabilized by bringing the anti-sway mechanism 26 into contact with the field F, and can measure the high-accuracy concentration p.

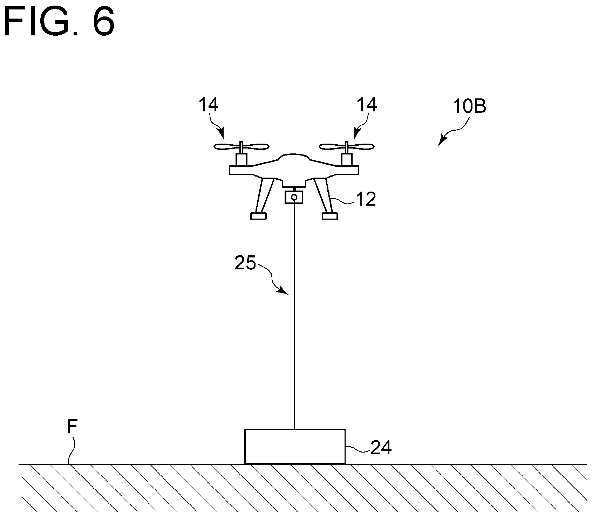

Further, in another mode of step S4 (concentration measurement step), the concentration measurement device 24 may measure the concentration p in a state in which the concentration measurement device 24 is lowered from the second UAV 10B to the field F by the hoisting and lowering mechanism 25. FIG. 6 is a schematic view showing, from the side, another mode of measuring the concentration p by the concentration measurement device 24 in step S3 of FIG. 3. In this mode, the concentration measurement device 24 is lowered to the field F which is the ground surface, thereby being allowed to measure the concentration p of the gas G at the gas leakage location on the ground surface.

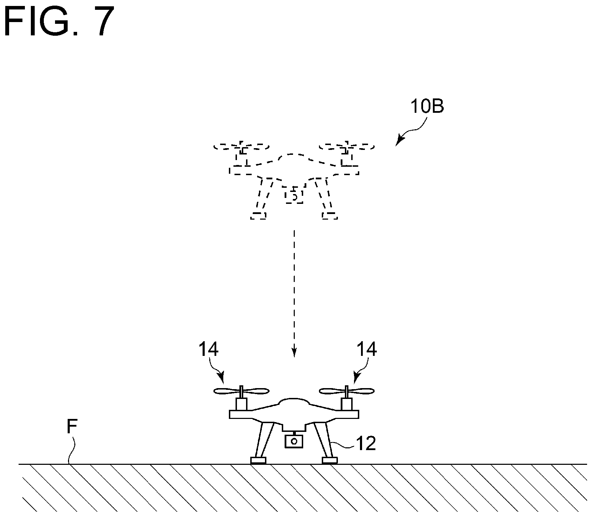

Further, in the another mode of step S4 (concentration measurement step), the concentration p at the predetermined height may be measured by lowering the second UAV 10 B mounted with the concentration measurement device 24. FIG. 7 is a schematic view showing, from the side, another mode of measuring the concentration p by the concentration measurement device 24 in step S3 of FIG. 3. In this mode, the second UAV 10B does not include the aforementioned hoisting and lowering mechanism 25, and the concentration p of the gas G at the predetermined height can be measured by lowering the second UAV 10B to the predetermined height, in a state in which the concentration measurement device 24 is stored in the UAV body 12.

Subsequently, returning to FIG. 3, the leakage amount calculation part 39 calculates the leakage amount Q of the gas G based on the volume V and the concentration p of the gas G detected in step S1 (step S5: leakage amount calculation step). In step S5, the volume V is estimated based on the spread of the gas G, which is identified from the image data of the field F, which is acquired as the detection result of the gas detection device in step S1, for example. Further, the measurement result in step S4 is used for the concentration p. Then, the leakage amount Q is calculated based on the aforementioned expression (1).

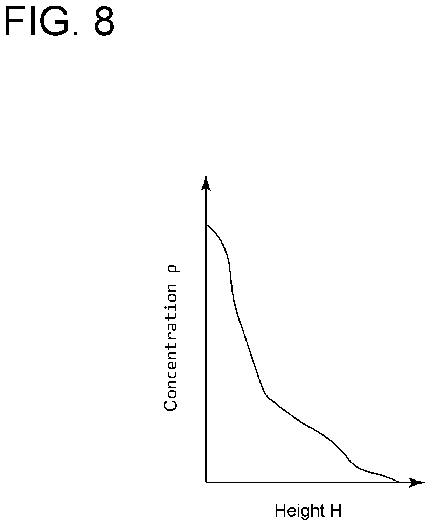

Further, in another mode of step S4 (concentration measurement step), when the concentration measurement device 24 is lowered from the second UAV 10B as shown in FIGs. 4 to 6 or when the second UAV 10B mounted with the concentration measurement device 24 is lowered as shown in FIG. 7, the concentration p may be measured at a plurality of different positions in the height direction during the lowering of the second UAV 10B. In this mode, a concentration distribution with respect to the height can be acquired by measuring a plurality of concentrations p at different heights. FIG. 8 is a graph showing an example of the concentration distribution with respect to the height.

Then, in step S5 (leakage amount calculation step), the concentration p at the gas leakage location may be estimated based on the concentration distribution of the gas G, which is thus acquired. By thus estimating the concentration p based on the concentration distribution, the leakage amount Q can be calculated using the higher-accuracy concentration $\rho$.

As described above, according to each of the above embodiments, the first UAV 10A capable of moving in the field F detects the gas G leaking in the field F and acquires the location information of the gas G (i.e., the gas leakage location). The location information acquired by the first UAV 10A is used by the second UAV 10B, causing the second UAV 10B to move to the gas leakage location. Then, the second UAV 10B measures the concentration of the gas G at the gas leakage location by using the mounted concentration measurement device 24. Further, the volume V of the gas G can be estimated based on the image data, etc., used to detect the gas G, for example. Based on the volume V and the concentration p of the gas G, which are thus obtained, the leakage amount Q of the gas G in the field F can be obtained, making it possible to quantitatively evaluate the gas leakage in the field F.

The contents described in the above embodiments would be understood as follows, for instance.

(1) A gas leakage monitoring method according to one aspect, includes: a detection step of detecting a gas leaking from a field, by using a gas detection device mounted on a first UAV; a location information acquisition step of acquiring location information of the gas by using a location measurement device mounted on the first UAV; a movement step of moving a second UAV mounted with a concentration measurement device including a contact gas sensor to above a gas leakage location corresponding to the location information; a concentration measurement step of measuring a concentration of the gas at the gas leakage location by using the concentration measurement device; and a leakage amount calculation step of calculating a leakage amount of the gas based on a volume of the gas detected by the gas detection device and the concentration measured by the concentration measurement device.

According to the above aspect (1), the first UAV capable of moving in the field detects the gas leaking in the field and acquires the location information of the gas (i.e., the gas leakage location). The location information acquired by the first UAV is used by the second UAV, causing the second UAV to move to the gas leakage location. Then, the second UAV measures the concentration of the gas at the gas leakage location by using the mounted concentration measurement device.

Further, the volume of the gas can be estimated based on the image data, etc., used to detect the gas, for example. Based on the volume and the concentration of the gas, which are thus obtained, the leakage amount of the gas in the field can be obtained, making it possible to quantitatively evaluate the gas leakage in the field.

In the present specification, "detection" is a concept including "sensing", and includes, for example, sensing the presence or absence of the gas leaking from the field, and detecting various parameters related to the gas.

(2) In another aspect, in the above aspect (1), the concentration measurement step includes causing the concentration measurement device to measure the concentration in a state in which the concentration measurement device is lowered from the second UAV to a predetermined height from the field.

According to the above aspect (2), the second UAV can preferably measure the concentration of the gas, which is used to calculate the leakage amount of the gas, by lowering the concentration measurement device from the second UAV to the predetermined height of the field, at the gas leakage location acquired by the first UAV.

(3) In another aspect, in the above aspect (2), the concentration measurement step includes lowering the concentration measurement device from the second UAV so that an anti-sway mechanism disposed at a bottom of the concentration measurement device contacts the field.

According to the above aspect (3), the anti-sway mechanism is disposed at the bottom of the concentration measurement device lowered from the second UAV. The anti-sway mechanism contacts the field when the height of the concentration measurement device lowered from the second UAV reaches the predetermined height, allowing the concentration measurement device to stably maintain its posture at the predetermined height.

(4) In another aspect, in the above aspect (1), the concentration measurement step includes causing the concentration measurement device to measure the concentration in a state in which the concentration measurement device is lowered from the second UAV to the field.

According to the above aspect (4), the second UAV can preferably measure the concentration of the gas, which is used to calculate the leakage amount of the gas, by lowering the concentration measurement device from the second UAV to the field, at the gas leakage location acquired by the first UAV.

(5) In another aspect, in the above aspect (1), the concentration measurement step includes measuring the concentration at the predetermined height by lowering the second UAV mounted with the concentration measurement device.

According to the above aspect (5), it is possible to preferably measure the concentration of the gas, which is used to calculate the leakage amount of the gas, by lowering the second UAV mounted with the concentration measurement device to the predetermined height, at the gas leakage location acquired by the first UAV.

(6) In another aspect, in the above aspect (1), the concentration measurement step includes causing the concentration measurement device to measure the concentration at a plurality of different positions in a height direction while being lowered from the second UAV, and the leakage amount calculation step includes estimating the concentration at the gas leakage location based on a concentration distribution of the gas.

According to the above aspect (6), the concentration distribution in the height direction is acquired by measuring the concentration of the gas at the different positions in the height direction, at the gas leakage location. The concentration of the gas at the gas leakage location can accurately be estimated by using the concentration distribution of the gas, which is thus acquired.

(7) A gas leakage monitoring system according to one aspect, includes: a first UAV mounted with a gas detection device for detecting a gas leaking from a field and a location measurement device for acquiring location information of the gas; a second UAV movable based on the location information and mounted with a concentration measurement device for measuring a concentration of the gas; and a leakage amount calculation device for calculating a leakage amount of the gas based on a volume of the gas detected by the gas detection device and the concentration measured by the concentration measurement device.

According to the above aspect (7), the first UAV capable of moving in the field detects the gas leaking in the field and acquires the location information of the gas (i.e., the gas leakage location). The location information acquired by the first UAV is used by the second UAV, causing the second UAV to move to the gas leakage location. Then, the second UAV measures the concentration of the gas at the gas leakage location by using the mounted concentration measurement device. Further, the volume of the gas can be estimated based on the image data, etc., used to detect the gas, for example. Based on the volume and the concentration of the gas, which are thus obtained, the leakage amount of the gas in the field can be obtained, making it possible to quantitatively evaluate the gas leakage in the field.

(8) In another aspect, in the above aspect (7), the leakage amount calculation device is disposed at a location remote from the first UAV and the second UAV, and receives data regarding the volume and the concentration via a wireless communication network.

According to the above aspect (8), since the various data can be acquired remotely via wireless communication from the first UAV and the second UAV movable in the field, in the remote location, it is possible to quantitatively monitor the gas leakage in the field.

(9) A gas leakage monitoring program according to one aspect, for causing a computer to execute: a detection step of detecting a gas leaking from a field, by using a gas detection device mounted on a first UAV; a location information acquisition step of acquiring location information of the gas by using a location measurement device mounted on the first UAV; a movement step of moving a second UAV mounted with a concentration measurement device including a contact gas sensor to above a gas leakage location corresponding to the location information; a concentration measurement step of measuring a concentration of the gas at the gas leakage location by using the concentration measurement device; and a leakage amount calculation step of calculating a leakage amount of the gas based on a volume of the gas detected by the gas detection device and the concentration measured by the concentration measurement device.

According to the above aspect (9), the first UAV capable of moving in the field detects the gas leaking in the field and acquires the location information of the gas (i.e., the gas leakage location). The location information acquired by the first UAV is used by the second UAV, causing the second UAV to move to the gas leakage location. Then, the second UAV measures the concentration of the gas at the gas leakage location by using the mounted concentration measurement device.

Further, the volume of the gas can be estimated based on the image data, etc., used to detect the gas, for example. Based on the volume and the concentration of the gas, which are thus obtained, the leakage amount of the gas in the field can be obtained, making it possible to quantitatively evaluate the gas leakage in the field.

Reference Signs List

1    Gas leakage monitoring system
12   Body
14   Propeller
20   Gas detection device
22   Location measurement device
24   Concentration measurement device
25   Hoisting and lowering mechanism
26   Anti-sway mechanism
30   Leakage amount calculation device
32   First UAV control part
34   Location information acquisition part
36   Second UAV control part
38   Concentration information acquisition part
39   Leakage amount calculation part
40   Wireless communication network

**Claims**

1. A gas leakage monitoring method, comprising:

   a detection step of detecting a gas leaking from a field, by using a gas detection device mounted on a first UAV;
   a location information acquisition step of acquiring location information of the gas by using a location measurement device mounted on the first UAV;
   a movement step of moving a second UAV mounted with a concentration measurement device including a contact gas sensor to above a gas leakage location corresponding to the location information;
   a concentration measurement step of measuring a concentration of the gas at the gas leakage location by using the concentration measurement device; and
   a leakage amount calculation step of calculating a leakage amount of the gas based on a volume of the gas detected by the gas detection device and the concentration measured by the concentration measurement device.

2. The gas leakage monitoring method according to claim 1,
   wherein the concentration measurement step includes causing the concentration measurement device to measure the concentration in a state in which the concentration measurement device is lowered from the second UAV to a predetermined height from the field.

3. The gas leakage monitoring method according to claim 2,
   wherein the concentration measurement step includes lowering the concentration measurement device from the second UAV so that an anti-sway mechanism disposed at a bottom of the concentration measurement device contacts the field.

4. The gas leakage monitoring method according to claim 1,
   wherein the concentration measurement step includes causing the concentration measurement device to measure the concentration in a state in which the concentration measurement device is lowered from the second UAV to the field.

5. The gas leakage monitoring method according to claim 1,
   wherein the concentration measurement step includes measuring the concentration at the predetermined height by lowering the second UAV mounted with the concentration measurement device.

6. The gas leakage monitoring method according to claim 1,

   wherein the concentration measurement step includes causing the concentration measurement device to measure the concentration at a plurality of different locations in a height direction while being lowered from the second UAV, and
   wherein the leakage amount calculation step includes estimating the concentration at the gas leakage location based on a concentration distribution of the gas.

7. A gas leakage monitoring system, comprising:

   a first UAV mounted with a gas detection device for detecting a gas leaking from a field and a location measurement device for acquiring location information of the gas;
   a second UAV movable based on the location information and mounted with a concentration measurement device for measuring a concentration of the gas; and
   a leakage amount calculation device for calculating a leakage amount of the gas based on a volume of the gas detected by the gas detection device and the concentration measured by the concentration measurement device.

8. The gas leakage monitoring system according to claim 7,
   wherein the leakage amount calculation device is disposed at a location remote from the first UAV and the second UAV, and receives data regarding the volume and the concentration via a wireless communication network.

9. A gas leakage monitoring program for causing a computer to execute:

   a detection step of detecting a gas leaking from a field, by using a gas detection device mounted on a first UAV;
   a location information acquisition step of acquiring location information of the gas by using a location measurement device mounted on the first UAV;
   a movement step of moving a second UAV mounted with a concentration measurement device including a contact gas sensor to above a gas leakage location corresponding to the location information;
   a concentration measurement step of measuring a concentration of the gas at the gas leakage location by using the concentration measurement device; and
   a leakage amount calculation step of calculating a leakage amount of the gas based on a volume of the gas detected by the gas detection device and the concentration measured by the concentration measurement device.

# FIG. 1

## FIG. 2

10A(10)

**First UAV**

| Gas detection device | ~20 |

| Location measurement device | ~22 |

10B(10)

**Second UAV**

| Concentration measurement device | ~24 |

40

**Leakage amount calculation device** ~30

| First UAV control part | ~32 |

| Location information acquisition part | ~34 |

| Second UAV control part | ~36 |

| Concentration information acquisition part | ~38 |

| Leakage amount calculation part | ~39 |

1

EP 4 553 468 A1

# FIG. 3

```
        ┌─────────────┐
        │    Start     │
        └──────┬──────┘
               │
               ▼
   ┌──────────────────────┐
   │ Detect gas by first  │ ─── S1
   │         UAV          │
   └──────────┬───────────┘
              │
              ▼
   ┌──────────────────────┐
   │ Acquire location     │ ─── S2
   │ information by first │
   │         UAV          │
   └──────────┬───────────┘
              │
              ▼
   ┌──────────────────────┐
   │   Move second UAV    │ ─── S3
   └──────────┬───────────┘
              │
              ▼
   ┌──────────────────────┐
   │ Measure concentration│ ─── S4
   └──────────┬───────────┘
              │
              ▼
   ┌──────────────────────┐
   │ Calculate leakage    │ ─── S5
   │       amount         │
   └──────────┬───────────┘
              │
              ▼
   ┌──────────────────────┐
   │ Calculate reference  │ ─── S6
   │ value (total movement│
   │ distance reference   │
   │       value)         │
   └──────────┬───────────┘
              │
              ▼
        ┌─────────────┐
        │     End      │
        └─────────────┘
```

~ S111

# FIG. 4

# FIG. 5

# FIG. 6

# FIG. 7

# FIG. 8

Concentration ρ

Height H

## INTERNATIONAL SEARCH REPORT

International application No.

**PCT/JP2023/006375**

### A. CLASSIFICATION OF SUBJECT MATTER

**G01M 3/02**(2006.01)i
FI: G01M3/02 L; G01M3/02 M

According to International Patent Classification (IPC) or to both national classification and IPC

### B. FIELDS SEARCHED

Minimum documentation searched (classification system followed by classification symbols)

G01M3/02

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| Y | US 2018/0292286 A1 (INTERNATIONAL BUSINESS MACHINES CORP.) 11 October 2018 (2018-10-11) paragraphs [0021]-[0024], [0032], [0036]-[0038], [0047], [0051] | 1-2, 4-9 |
| A | | 3 |
| Y | JP 2020-521115 A (SAUDI ARABIAN OIL CO.) 16 July 2020 (2020-07-16) paragraphs [0032], [0033], [0069] | 1-2, 4-9 |
| Y | US 4785658 A (JACKSON, John) 22 November 1988 (1988-11-22) column 5, line 64 to column 6, line 4 | 2, 4, 6 |
| A | WO 2017/022556 A1 (KONICA MINOLTA, INC.) 09 February 2017 (2017-02-09) | 1-9 |

☐ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **27 April 2023** | **16 May 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

INTERNATIONAL SEARCH REPORT
Information on patent family members

International application No.

**PCT/JP2023/006375**

| Patent document cited in search report | | | Publication date (day/month/year) | Patent family member(s) | | | Publication date (day/month/year) |
|---|---|---|---|---|---|---|---|
| US | 2018/0292286 | A1 | 11 October 2018 | US | 2018/0292374 | A1 | |
| JP | 2020-521115 | A | 16 July 2020 | US<br>paragraphs [0037], [0038],<br>[0074]<br>US<br>WO<br>KR<br>CN | 2018/0335404<br><br><br>2019/0086365<br>2018/212891<br>10-2020-0018454<br>110892259 | A1<br><br><br>A1<br>A1<br>A<br>A | |
| US | 4785658 | A | 22 November 1988 | (Family: none) | | | |
| WO | 2017/022556 | A1 | 09 February 2017 | US<br>EP | 2018/0222581<br>3315937 | A1<br>A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022138048 A
- JP 4266535 B